# EUROPÄISCHE PATENTANMELDUNG

(11) **EP 2 253 263 A1**
(43) Veröffentlichungstag der Anmeldung: **24.11.2010**
(21) Anmeldenummer: 09160504.8
(22) Anmeldetag: 18.05.2009
(51) Int. Cl.: A61B 1/04

(54) **Tragbares medizinisches Aufzeichnungsgerät**

(71) Anmelder: MB-Technics Gmbh, 85567 Grafing (DE)
(72) Erfinder: Bodenburg, Martin, 85567, Grafing (DE)
(74) Vertreter: TBK-Patent

(57) **Zusammenfassung**

Ein erfindungsgemäßes Aufzeichnungsgerät zur medizinischen, insbesondere zahnmedizinischen Anwendung ist beschrieben, das ein am Körper eines Benutzers tragbares Gerätegehäuse aufweist, wobei in dem Gerätegehäuse eine Batterie und eine Datenspeichereinheit enthalten sind. Eine Kamera zur Beobachtung eines Behandlungsfelds ist über eine Kabelverbindung mit der Datenspeichereinheit und der Batterie verbunden. Außerdem ist eine das Behandlungsfeld mit sichtbarem Licht ausleuchtende Stirnlampe über eine Kabelverbindung mit der Batterie verbunden. Darüber hinaus ist ein Mikrofon an dem Gerätegehäuse angeschlossen und mit der Datenspeichereinheit elektrisch verbunden. Die Kamera, die Stirnlampe und das Mikrofon sind über ein an dem Gerätegehäuse vorgesehenes Bedienfeld ansteuerbar.

## Beschreibung

### TECHNISCHES GEBIET

Die Erfindung bezieht sich auf ein Aufzeichnungsgerät, und genauer gesagt auf ein Aufzeichnungsgerät zur medizinischen, insbesondere zahnmedizinischen Anwendung, das ein am Körper eines Benutzers tragbares Gerätegehäuse hat, in dem u.a. eine Batterie und eine Datenspeichereinheit enthalten sind.

### STAND DER TECHNIK

Auf dem Gebiet der Medizin, insbesondere auf dem Gebiet der Zahnmedizin ist es bisher bekannt, ein mobiles LED-Lichtsystem ohne externe Verkabelungen zu verwenden, das dem Benutzer, d.h. dem behandelnden Arzt, ein freies und von stationären Strom - bzw. Lichtquellen unabhängiges Arbeiten ermöglicht. Als Beispiel für ein derartiges LED-Lichtsystem ist an dieser Stelle das starLight^{nano}® der Fa. starMed Technik genannt, bei dem eine LED-Lampe über eine Stromkabelverbindung mit einem Gerätehauptkörper verbunden ist, in dem ein abnehmbarer Lithium-Kobalt-Akkumulator vorgesehen ist. Der LED-Lampenkörper ist in der Regel an einer von dem Arzt getragenen Lupenbrille angebracht, um diesem bei der Behandlung eines Patienten freie Hände und ausreichende Beleuchtung im Behandlungsfeld zu ermöglichen.

In den letzten Jahren hat es sich davon abgesehen jedoch herausgestellt, dass es für Ärzte immer wichtiger wird, die Behandlung bzw. die Diagnose eines Patienten möglichst einfach dokumentieren zu können.

### ZUSAMMENFASSUNG DER ERFINDUNG

Der Erfindung liegt daher die Aufgabe zugrunde, ein tragbares Aufzeichnungsgerät bereitzustellen das von einem Benutzer, i.d.R. einem Arzt zur Dokumentation beispielsweise während der Behandlung oder während der Diagnose des Patienten verwendet werden kann. Diese Aufgabe wird erfindungsgemäß durch ein Aufzeichnungsgerät mit den Merkmalen des Anspruchs 1 gelöst.

Ein erfindungsgemäßes Aufzeichnungsgerät zur medizinischen, insbesondere zahnmedizinischen Anwendung, weist ein am Körper tragbares Gerätegehäuse auf, in dem eine Batterie und eine Datenspeichereinheit enthalten sind. Bei dem erfindungsgemäßen Aufzeichnungsgerät ist zudem eine Kamera zur Beobachtung bzw. zur Aufzeichnung eines Behandlungsfelds über eine Kabelverbindung mit der Datenspeichereinheit und der Batterie verbunden. Die Kamera hat vorzugsweise einen Brennweitenbereich bis 1000mm, weiter vorzugsweise bis 600mm, was in der Regel dem maximalen üblichen Abstand des Arzts zum Behandlungsfeld entspricht.

Des Weiteren ist eine Stirnlampe über eine Kabelverbindung mit der Batterie verbunden, wobei die Stirnlampe das Behandlungsfeld mit sichtbarem Licht ausleuchtet. Die Stirnlampe hat dabei vorzugsweise eine Beleuchtungsstärke von mindestens 10.000 Lux, um dem Arzt eine ausreichende Beleuchtung des Behandlungsfelds zu ermöglichen, und kann eine LED-Lampe oder dergleichen sein.

Es ist bei dem erfindungsgemäßen Aufzeichnungsgerät denkbar, dass die Kamera und die Stirnlampe in einem Bauteil integriert sind, wobei die jeweilige Kabelverbindung der Kamera und der Stirnlampe durch eine gemeinsame Kabelverbindung verwirklicht wird. Darüber hinaus kann die Kamera eine CMOS-Kamera, eine CCD-Kamera oder dergleichen sein und die Linse der Kamera kann mit einer schmutzabweisenden Lotuseffekt-Beschichtung versehen sein, wie z.B. der Zeiss LotuTec®-Beschichtung.

Darüber hinaus ist ein Mikrofon an dem Gerätegehäuse angeschlossen und mit der Datenspeichereinheit elektrisch verbunden. Bei dem erfindungsgemäßen Aufzeichnungsgerät ist das Mikrofon vorzugsweise in dem Gerätegehäuse integriert und nach Art eines Diktiergeräts durch eine Einhandbedienung betätigbar. Das bedeutet, dass der Arzt das Gerätegehäuse mit der Hand vor den Mund führen und mit dem Daumen einen oder mehrere Knöpfe bedienen kann, durch deren Betätigung das Mikrofon und die Aufnahme gestartet und gestoppt sowie eine bereits gemachte Aufzeichnung vorwärts oder rückwärts gespult und abgespielt werden kann.

Die Kamera, die Stirnlampe und das Mikrofon sind über ein an dem Gerätegehäuse vorgesehenes Bedienfeld einzeln oder gemeinsam ansteuerbar. Das Bedienfeld kann verschiedene Tasten, wie z.B. eine Start-/Stop-Taste, eine Vorwärts/Rückwärts-Taste, usw., oder auch ein einzelnes Multifunktionselement aufweisen, das beispielsweise gedreht und in verschiedene Richtungen gedrückt und gezogen werden kann. Derartige Elemente sind auch als Joystick bekannt. Darüber hinaus ist es denkbar, dass das Bedienfeld ein Touchscreen-Bedienfeld ist, auf dem die benötigten Bedientasten je nach Anwendung virtuell dargestellt werden und von dem Arzt durch Drücken des entsprechenden Bereichs des Touchscreen-Bedienfelds bedient werden können.

Weiterhin vorzugsweise ist ein Lautsprecher in dem Gerätegehäuse integriert, der dazu dienen kann, Warntöne auszugeben oder das Diktat des Arztes wiederzugeben. Außerdem ist vorzugsweise ein Bildschirm in dem Gerätegehäuse integriert, mit dem der Arzt beispielsweise erkennen kann, ob die Kamera ein scharfes Bild aufnimmt, oder auf dem der Arzt das von der Kamera bereits aufgenommene Bildmaterial ansehen kann. Der Bildschirm kann beispielsweise ein LC-Bildschirm oder dergleichen sein und kann u.a. als Überwachungsanzeige für das Diktat dienen, wobei z.B. die bereits gesprochene Minutenzahl angezeigt werden kann.

Das Gerätegehäuse kann des Weiteren vorzugsweise eine Schnittstellengruppe zur Verbindung der Datenspeichereinheit nach außerhalb des Gerätegehäuses aufweisen. Die Schnittstellengruppe kann dabei einen Anschluss für ein serielles Bussystem, wie z.B. einen USB-Anschluss, einen Micro-USB-Anschluss oder dergleichen, oder eine kabellose Übertragungseinrichtung zur Ausgabe von Daten an beispielsweise einen Computer aufweisen, wie z.B. eine WLAN-Übertragungseinrichtung, eine Bluetooth-Übertragungseinrichtung, eine Infrarot-

Übertragungseinrichtung oder dergleichen. Des Weiteren ist es denkbar, dass die Schnittstellenbaugruppe einen AV-Ausgang umfasst, der dazu dient, das durch die Kamera aufgezeichnete Bildmaterial über einen anzuschließenden Bildschirm wiederzugeben.

Die Datenspeichereinheit des erfindungsgemäßen Aufzeichnungsgeräts weist vorzugsweise eine Schreib-/Leseeinheit und ein Speichermedium auf. Dabei kann das Gerätegehäuse derart gestaltet sein, dass ein Einsetzen und Entnehmen des Speichermediums ohne große Umstände möglich ist, wobei das Speichermedium eine elektronische Speicherkarte sein kann, wie z.B. eine SD-Karte, eine Micro-SD-Karte oder dergleichen. Dabei ist es denkbar, dass die Speicherkarte von außerhalb des Gerätegehäuses zugänglich in einen entsprechenden Steckplatz in dem Gerätegehäuse, der als Schreib-/Leseeinheit der Datenspeichereinheit dient, einsteckbar und aus diesem wieder herausnehmbar ist.

Bei dem erfindungsgemäßen Aufzeichnungsgerät ist die Batterie vorzugsweise derart in dem Gerätegehäuse vorgesehen, dass diese auswechselbar ist und beispielsweise durch eine Ersatzbatterie ersetzt werden kann. Des Weiteren ist es vorzuziehen, dass die Batterie aufladbar ist. Dabei ist es denkbar, dass die Schnittstellengruppe eine Verbindung der Batterie nach außerhalb des Gerätegehäuses bietet, wie z.B. eine Ladebuchse, um ein Laden der Batterie zu ermöglichen.

Die Stirnlampe oder die Kamera oder beide können vorzugsweise an einer Lupenbrille oder einem Kopfband angebracht sein, die bzw. das der Arzt während der Behandlung trägt. Dabei ist es weiter vorzuziehen, dass das Gerätegehäuse mit dem daran angeschlossenen Komponenten an der Kleidung bzw. am Körper des Arztes befestigt werden kann, beispielsweise dadurch, dass eine Gürtelklammer an dem Gerätegehäuse angebracht ist, mit der das Gerätegehäuse an einem Gürtel des Arztes befestigt werden kann, oder dadurch, dass das Gerätegehäuse selbst einen Schenkel einer Federklammer bildet, mit der das Gerätegehäuse an dem Gürtel oder der Kleidung des Arztes angeklemmt werden kann.

Es ist ferner weiterhin denkbar, dass eine Digitalanzeige zur Funktionsüberwachung beispielsweise der Kamera und/oder des Mikrofons bzw. der Diktierfunktion und/oder eine oder mehrere Warnsignallampen in dem Gerätegehäuse integriert sind. Die Warnsignallampen können dabei dazu dienen, den Arzt auf eine Fehlfunktion der Kamera, der Datenspeichereinheit oder dergleichen aufmerksam zu machen.

### KURZBESCHREIBUNG DER ZEICHNUNGEN

Fig. 1 zeigt eine Vorderansicht des Aufzeichnungsgeräts gemäß einem Ausführungsbeispiel der vorliegenden Erfindung mit daran angeschlossener Kamera und Stirnlampe;
Fig. 2a zeigt eine Vorderansicht eines Gerätegehäuses des in Fig. 1 gezeigten Aufzeichnungsgeräts ohne daran angeschlossene Komponenten;
Fig. 2b zeigt eine Rückansicht des in Fig. 2a gezeigten Gerätegehäuses;
Fig. 2c zeigt eine Draufsicht des in Fig. 2a gezeigten Gerätegehäuses aus Sicht des Pfeils I in Fig. 2b; und
Fig. 2d zeigt eine Seitenansicht des in Fig. 2a gezeigten Gerätegehäuses aus Sicht des Pfeils II in Fig. 2b.

### AUSFÜHRLICHE BESCHREIBUNG EINES AUSFÜHRUNGSBEISPIELS

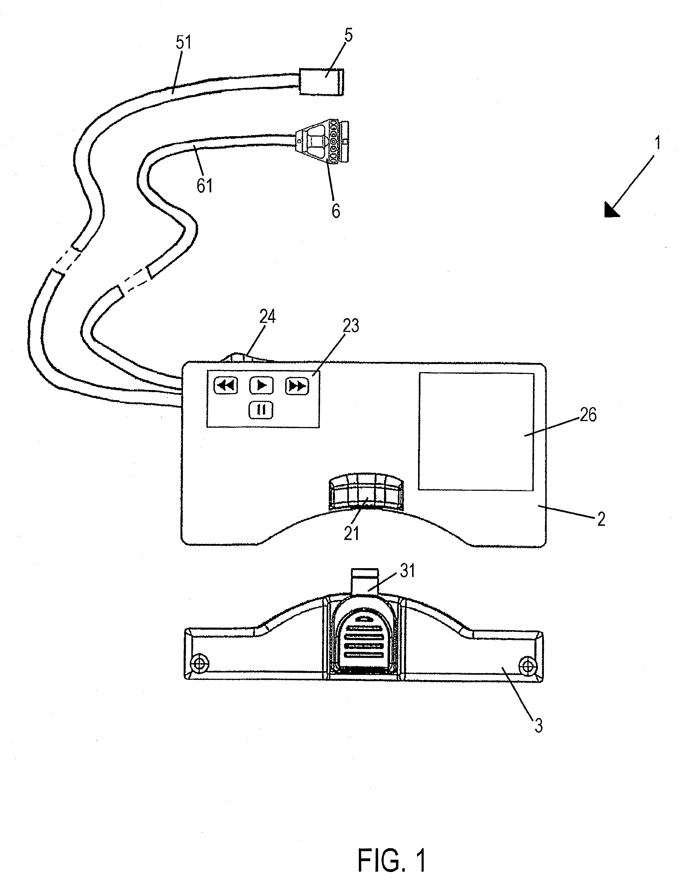

Fig. 1 zeigt eine Vorderansicht des Aufzeichnungsgeräts 1 gemäß einem Ausführungsbeispiel der Erfindung. Das Aufzeichnungsgerät 1 ist dabei mit von einem Gerätegehäuse 2 abgetrennter Batterie 3 gezeigt. Das Gerätegehäuse 2 weist eine viereckige Form auf, wobei eine halbrunde Aussparung in dem Gerätegehäuse 2 vorgesehen ist, die einem halbrunden Vorsprung der Batterie 3 entspricht. Das Gerätegehäuse 2 sowie ein Gehäuse der Batterie 3 sind aus Kunststoff spritzgegossen.

Eine Kamera 5 ist über eine Kabelverbindung 51 und eine Stirnlampe 6 ist über eine andere Kabelverbindung 61 mit dem Gerätegehäuse 2 bzw. mit den in dem Gerätegehäuse 2 angeordneten zugehörigen Bauteilen fest verbunden. Die Kabelverbindungen 51 und 61 können alternativ dazu lösbar mit dem Gerätegehäuse 2 verbunden sein. Die Batterie 3 kann durch ein an beiden Seiten der Batterie 3 vorgesehenes Steckelement 31 an entsprechend an dem Gerätegehäuse 2 vorgesehene Steckanschlüsse 21 angesteckt und dadurch mit dem Gerätegehäuse 2 abnehmbar verbunden werden.

Wie es in Fig. 1 weiter zu sehen ist, sind in bzw. an dem Gerätegehäuse 2 ein Bedienfeld 23, ein Einhandbedienelement 24 für die Diktiergerätefunktion des Aufzeichnungsgeräts 1 sowie ein Bildschirm 26 vorgesehen. Die Elemente 23, 24 und 26 sind für sich selbstständige Bauteile, die in entsprechenden Aussparungen des Gerätegehäuses 2 angeordnet und mit diesem fest verbunden sind.

Der Bildschirm 26 dient beispielsweise dazu, ein von der Kamera 5 gesendetes Bild anzuzeigen, um eine richtige Justierung der Kamera 5 zu überprüfen. Außerdem kann mit dem Bildschirm 26 bereits aufgezeichnetes Bildmaterial gesichtet werden. Durch verschiedene, in dem Bedienfeld 23 vorgesehene Bedientasten kann das auf dem Bildschirm 26 angezeigte Bildmaterial entsprechend abgespielt, angehalten, vor- und zurückgespult werden.

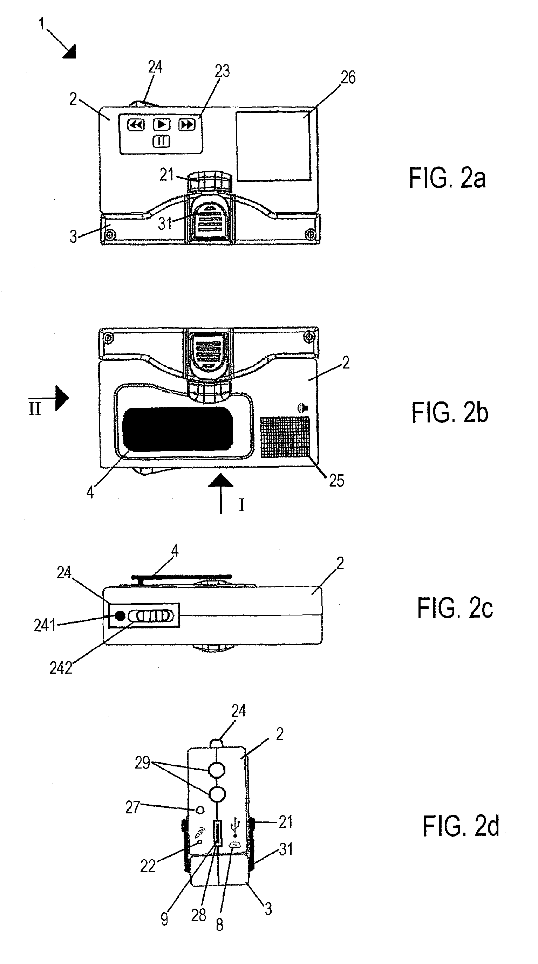

Figuren 2a bis 2d zeigen das Aufzeichnungsgerät 1 des Ausführungsbeispiels in verschiedenen Ansichten, jedoch ohne Kamera 5 und Stirnlampe 6.

Fig. 2a zeigt eine Vorderansicht des Aufzeichnungsgeräts 1 des Ausführungsbeispiels, wobei die Batterie 3 in dieser Ansicht über die Steckverbindung 21, 31 mit dem Gerätegehäuse 2 verbunden ist.

Fig. 2b zeigt eine Rückansicht des Aufzeichnungsgeräts 1 des Ausführungsbeispiels, das auch in Fig. 2a gezeigt ist.

Eine Gürtelklammer 4 ist an der Rückseite des Gerätegehäuses 2 befestigt, durch die das Gerätegehäuse 2 an einem Gürtel (nicht gezeigt) eines Benutzers des Aufzeichnungsgeräts 1 angeklemmt werden kann. Darüber hinaus ist ein Lautsprecher 25 an der Rückseite des Gerätegehäuses 2 vorgesehen.

Fig. 2c ist eine Draufsicht des Aufzeichnungsgeräts 1 des Ausführungsbeispiels aus der Richtung des Pfeils I in Fig. 2b. In dieser Ansicht ist klar zu erkennen, dass das Einhandbedienelement 24 wie bei herkömmlichen Diktiergeräten üblich aus einem Druckknopf 241 und einem Schieber 242 besteht, der vor- und zurückgeschoben werden kann und dementsprechend verschiedene Vorgänge ansteuert, wie z.B. eine Wiedergabe des Diktats durch den Lautsprecher 25, ein Vor- oder Zurückspielen des Diktats, usw.

Fig. 2d ist eine Seitenansicht der in Fig. 2b gezeigten Ansicht des Aufzeichnungsgeräts 1 aus der Richtung des Pfeils II. An der gezeigten Seite des Aufzeichnungsgeräts 1 sind in dem Ausführungsbeispiel die Kamera 5 und die Stirnlampe 6 an den Anschlüssen 29 des Gerätegehäuses 2 angeschlossen. Darüber hinaus sind an dieser Seite des Aufzeichnungsgeräts 1 des Weiteren eine Warnlampe 27, ein Mikrofon 22 als Aufnahmeelement für die Diktiergerätefunktion des Aufzeichnungsgeräts 1, ein Micro-USB-Anschluss 8 als serielle Schnittstelle sowie eine Öffnung einer Schreib-/Leseeinheit 28 mit eingesetzter Micro-SD-Karte 9 gezeigt.

### ABWANDLUNGEN DES AUSFÜHRUNGSBEISPIELS

Die Erfindung ist nicht auf das vorstehende ausführliche Ausführungsbeispiel beschränkt.

Alternativ zur vorhergehend beschriebenen Mikro-SD-Karte 9 können andere gängige Speichermedien in die Schreib-/Leseeinheit 28 eingesetzt werden, wie z.B. eine SD-Karte, eine Multimedia-Karte, und dergleichen.

Alternativ zum Micro-USB-Anschluss 8 kann jede andere Anschlussart in dem Gerätegehäuse 2 vorgesehen sein, wie z.B. ein USB-Anschluss, ein Firewire-Anschluss, sowie jede gängige Art von drahtloser Übertragungseinrichtung, wie z.B. eine Bluetooth-Übertragungseinrichtung, eine WLAN-Übertragungseinrichtung und dergleichen.

Der Lautsprecher 25 kann als Alternative zu dem vorhergehend beschriebenen Ausführungsbeispiel alternativ auch an einer anderen Stelle an dem Gerätegehäuse 2 vorgesehen sein; beispielsweise an einer Oberseite des Gerätegehäuses, an der die Kamera 5 und die Stirnlampe 6 angeschlossen sind und die in einem am Gürtel des Benutzers festgeklemmten Zustand des Aufzeichnungsgeräts 1 zum Oberkörper des Benutzers hin gerichtet ist.

## Patentansprüche

1. Aufzeichnungsgerät (1) zur medizinischen, insbesondere zahnmedizinischen Anwendung, mit einem am Körper tragbaren Gerätegehäuse (2), in dem eine Batterie (3) und eine Datenspeichereinheit (28) enthalten sind, wobei
eine Kamera (5) zur Beobachtung eines Behandlungsfelds über eine Kabelverbindung (51) mit der Datenspeichereinheit (28) und der Batterie (3) verbunden ist,
eine das Behandlungsfeld mit sichtbarem Licht ausleuchtende Stirnlampe (6) über eine Kabelverbindung (61) mit der Batterie (3) verbunden ist,
ein Mikrofon (22) an dem Gerätegehäuse (2) angeschlossen und mit der Datenspeichereinheit (28) elektrisch verbunden ist, und
die Kamera (5), die Stirnlampe (6) und das Mikrofon (22) über ein an dem Gerätegehäuse (2) vorgesehenes Bedienfeld (23, 24) ansteuerbar sind.

2. Aufzeichnungsgerät (1) nach Anspruch 1, wobei das Mikrofon (22) in dem Gerätegehäuse (2) integriert und nach Art eines Diktiergeräts durch eine Einhandbedienung (24) betätigbar ist.

3. Aufzeichnungsgerät (1) nach einem der vorangehenden Ansprüche, wobei des Weiteren ein Lautsprecher (25) und/oder ein Bildschirm (26) in dem Gerätegehäuse (2) integriert ist.

4. Aufzeichnungsgerät (1) nach einem der vorangehenden Ansprüche, wobei die Stirnlampe (6) eine Beleuchtungsstärke von mindestens 10.000Lux hat.

5. Aufzeichnungsgerät (1) nach einem der vorangehenden Ansprüche, wobei die Kamera (5) einen Brennweitenbereich bis 600mm hat, der dem Abstand des Benutzers zum Behandlungsfeld entspricht.

6. Aufzeichnungsgerät (1) nach einem der vorangehenden Ansprüche, wobei die Datenspeichereinheit (28) eine Schreib-/Leseeinheit und ein Speichermedium (7) aufweist und wobei das Gerätegehäuse (2) angepasst ist, um ein Einsetzen und Entnehmen des Speichermediums (7) zu ermöglichen.

7. Aufzeichnungsgerät (1) nach Anspruch 6, wobei das Speichermedium (7) eine elektronische Speicherkarte (7) ist.

8. Aufzeichnungsgerät (1) nach einem der vorangehenden Ansprüche, wobei die Batterie (3) in dem Gerätegehäuse (2) auswechselbar vorgesehen ist und/oder aufladbar ist.

9. Aufzeichnungsgerät (1) nach einem der vorangehenden Ansprüche, wobei das Gerätegehäuse (2) eine Schnittstellengruppe (8) zur Verbindung der Datenspeichereinheit (7) und/oder der Batterie (3) nach außerhalb des Gerätegehäuses (2) aufweist.

10. Aufzeichnungsgerät (1) nach Anspruch 9, wobei die
Schnittstellengruppe (8) eine Ladebuchse zum Laden der Batterie und/oder einen Anschluss für ein serielles Bussystem (8) und/oder eine kabellose Übertragungseinrichtung umfasst.

11. Aufzeichnungsgerät (1) nach einem der vorangehenden
Ansprüche, wobei die Stirnlampe (6) und/oder die Kamera (5) an einer Lupenbrille oder einem Kopfband angebracht ist.

12. Aufzeichnungsgerät (1) nach einem der vorangehenden
Ansprüche, wobei eine Digitalanzeige zur Funktionsüberwachung und/oder eine Warnsignallampe (27) in dem Gerätegehäuse (2) integriert ist.

13. Aufzeichnungsgerät (1) nach einem der vorangehenden
Ansprüche, wobei das Gerätegehäuse (2) an der Kleidung bzw. am Körper eines Benutzers befestigbar ist.

14. Aufzeichnungsgerät (1) nach Anspruch 13, wobei eine
Gürtelklammer (4) an dem Gerätegehäuse (2) angebracht ist oder das Gerätegehäuse (2) selbst einen Schenkel einer Federklammer bildet.

15. Aufzeichnungsgerät (1) nach einem der vorangehenden
Ansprüche, wobei die Kameralinse mit einer schmutzabweisenden Lotuseffekt-Beschichtung versehen ist.
